# EUROPEAN PATENT APPLICATION

(11) **EP 4 487 796 A1**
(43) Date of publication of application: **08.01.2025**
(21) Application number: 23758940.3
(22) Date of filing: 19.01.2023
(51) Int. Cl.: A61B 17/3203

(54) **MEDICAL WATER JET SCALPEL DEVICE AND MEDICAL WATER JET SCALPEL SYSTEM**

(30) Priority: 28.02.2022 CN 202210188029
(71) Applicant: Bluesail Surgical Co., Ltd., Shanghai 201321 (CN)
(72) Inventor: DING, Weijiang, Shanghai 201321 (CN); YANG, Shenghua, Shanghai 201321 (CN)
(74) Representative: Viering, Jentschura & Partner mbB Patent- und Rechtsanwälte
(86) International application number: PCT/CN2023/073127
(87) International publication number: WO 2023/160315

(57) **Abstract**

A medical water jet scalpel device (100) and a medical water jet scalpel system are disclosed. The medical water jet scalpel system comprises the medical water jet scalpel device (100). The medical water jet scalpel device (100) comprises an outer tube (110), a jet tube (120), a nozzle (130) and a driving apparatus (140). The jet tube (120) extends through the outer tube (110) and is configured to input medical liquid. A gap is formed between an outer wall of the jet tube (120) and an inner wall of the outer pipe (110). The nozzle (130) is arranged at a distal end of the jet tube (120) and is configured to output the medical liquid. The driving apparatus (140) is connected to at least part of the jet tube (120) and is configured to drive the distal end of the jet tube (120) to swing back and forth or rotate around the central axis of the outer tube (110). The medical water jet scalpel device (100) automatically drives, by means of the driving apparatus (140), the jet tube (120) limited in the outer tube (110) to move, such that the nozzle (130) of the medical water jet scalpel device (100) performs a rapid scanning motion to replace rapid shaking of a surgeon when manually operating the medical water jet scalpel device (100), thereby greatly reducing the operation difficulty, increasing the efficiency of a surgical operation for selective separation by a water jet scalpel, and improving the effect of tissue selective separation and the surgical operation accuracy.

## Description

### CROSS-REFERENCE TO RELATED APPLICATION(S)

The present application claims priority to Chinese patent application No. 202210188029.1 filed on February 28, 2022, the disclosure of which is incorporated herein by reference in its entirety as part of the present application, for all purposes.

### TECHNICAL FIELD

The present disclosure relates to a medical water jet scalpel device and a medical water jet scalpel system.

### BACKGROUND

At present, cancer is one of the major diseases that have not been conquered by modern medicine. According to some cancer statistics, liver cancer ranks the fifth in incidence and the second in mortality among various cancer diseases. The high incidence and mortality of liver cancer reflect the fact that there is still absent of effective and targeted therapeutic methods currently.

Therapeutic methods for liver cancer include surgical treatment, local ablation, transcatheter arterial chemoembolization, radiation therapy, systemic therapy and the like, among which surgical treatment remains as a preferred one for liver cancer.

The key operating steps of a surgical treatment include dissociation of liver from ligaments, blocking of inflow of blood vessels and bile ducts (to the first hepatic portal), liver resection, blocking of outflow of blood vessels and bile ducts (to the second hepatic portal and the third hepatic portal), and abdominal closure after complete hemostasis. However, a surgery may also lead to complications such as infection, bleeding, bile leakage, and liver failure, which has seriously affected the five-year survival rate and the mortality rate of liver cancer. From the aspects of operation difficulty, time consumption and influence on complications of these key steps, liver resection is the most noteworthy.

At present, surgical treatments for liver resection are mainly divided into three categories, and there are more than ten specific methods. For example, the commonly used methods include: (1) separation of liver parenchyma and preservation of blood vessels; (2) separation of liver parenchyma, and closure and cutting of blood vessels and bile ducts; (3) coagulation of resected surface of liver parenchyma, and cutting of the same with a scalpel.

### SUMMARY

At least one embodiment of the present disclosure provides a medical water jet scalpel device, which includes an outer tube, a water jet tube, a water jet nozzle and a driving device. The water jet tube extends through the outer tube and is configured to input medical liquid, and a gap is provided between an outer wall of the water jet tube and an inner wall of the outer tube. The water jet nozzle is arranged at a distal end of water jet tube and configured to output the medical liquid. The driving device is connected with at least part of the water jet tube, and configured to drive a distal end part of the water jet tube to swing reciprocally or revolve around a central axis of the outer tube.

For example, in the medical water jet scalpel device provided by at least one embodiment of the present disclosure, a proximal end part of the water jet tube includes a connecting part fixedly connected with at least part of the driving device, the distal end part of the water jet tube and the connecting part of the water jet tube are not coaxial with each other, so that the driving device drives the proximal end part of the water jet tube to rotate around a central axis of the water jet tube and enables the distal end part of the water jet tube to revolve around the central axis of the outer tube.

For example, in the medical water jet scalpel device provided by at least one embodiment of the present disclosure, the water jet tube is integrally bent so that the distal end part of the water jet tube and the connecting part of the water jet tube are not coaxial with each other.

For example, in the medical water jet scalpel device provided by at least one embodiment of the present disclosure, the distal end part of the water jet tube is configured as a bending part, and the distal end part of the water jet tube has at least two segments which are not coaxial with each other, so that the distal end part of the water jet tube and the connecting part of the water jet tube are not coaxial with each other.

For example, in the medical water jet scalpel device provided by at least one embodiment of the present disclosure, when the distal end part of the water jet tube abuts against an inner wall of a first side of the outer tube along a radial direction, the proximal end part of the water jet tube abuts against an inner wall of the other side of the outer tube opposite to the first side along the radial direction.

For example, in the medical water jet scalpel device provided by at least one embodiment of the present disclosure, when the distal end part of the water jet tube abuts against an inner wall of a first side of the outer tube along a radial direction, the proximal end part of the water jet tube is arranged with a gap relative to an inner wall of the other side of the outer tube opposite to the first side along the radial direction.

For example, in the medical water jet scalpel device provided by at least one embodiment of the present disclosure, the proximal end part of the water jet tube is configured to be fixed relative to the outer tube, and the driving device is configured to drive the distal end part of the water jet tube to swing reciprocally in one dimension along a radial direction of the water jet tube relative to the outer tube, or to swing circumferentially in two dimensions around the central axis of the outer tube.

For example, the medical water jet scalpel device provided by at least one embodiment of the present disclosure further includes a supporting body, wherein the supporting body is sleeved outside a proximal end of the outer tube, and the supporting body is fixedly connected with the proximal end of the outer tube.

For example, in the medical water jet scalpel device provided by at least one embodiment of the present disclosure, the supporting body includes a handle, the handle is fixedly connected with the driving device, and the handle is configured to be driven by an external driving source to move in a direction of the central axis of the outer tube, so that the distal end part of the water jet tube moves in the direction of the central axis of the outer tube.

For example, in the medical water jet scalpel device provided by at least one embodiment of the present disclosure, the driving device includes a driving motor, and the driving motor is connected with the connecting part of the water jet tube to drive the proximal end part of the water jet tube to rotate around the central axis of the water jet tube.

For example, in the medical water jet scalpel device provided by at least one embodiment of the present disclosure, the handle includes a shell, the driving motor includes a first motor and the first motor is arranged in the shell; the driving device further includes a driver connecting line, and the first motor is configured to obtain electric energy and a control signal through the driver connecting line; and the first motor is sleeved outside the connecting part of the water jet tube and coaxially fixed with the water jet tube to drive the proximal end part of the water jet tube to rotate around the central axis of the water jet tube.

For example, in the medical water jet scalpel device provided by at least one embodiment of the present disclosure, the handle includes a shell, the driving motor includes a second motor, the driving device further includes a first connector, and the second motor and the first connector are arranged in the shell of the handle. The first connector includes a first gear and a second gear, and the first gear is meshed with the second gear; the first gear is fixedly connected with the connecting part of the water jet tube, and the second gear is connected with the second motor; and the second motor is configured to drive the second gear to rotate to drive the first gear to rotate, so as to drive the proximal end part of the water jet tube to rotate around the central axis of the water jet tube.

For example, in the medical water jet scalpel device provided by at least one embodiment of the present disclosure, the first connector includes at least one of the following: a gear, a belt pulley and a connecting rod.

For example, the medical water jet scalpel device provided by at least one embodiment of the present disclosure further includes a liquid supply connecting pipeline, wherein one end of the liquid supply connecting pipeline is connected with a proximal end of the water jet tube, and the other end of the liquid supply connecting pipeline is configured to be connected with an output end of a pressure pump to input the medical liquid.

For example, in the medical water jet scalpel device provided by at least one embodiment of the present disclosure, the handle includes a shell, the driving motor is arranged outside the shell, and the driving motor is fixedly connected with the liquid supply connecting pipeline and configured to drive the liquid supply connecting pipeline to rotate so as to drive the proximal end part of the water jet tube to rotate around the central axis of the water jet tube.

For example, the medical water jet scalpel device provided by at least one embodiment of the present disclosure further includes a supporting body, wherein the supporting body includes a handle, and the handle includes a shell, wherein the shell of the handle is sleeved outside a proximal end of the outer tube and fixedly connected with the proximal end of the outer tube. The driving device includes an electromagnetic ring and a magnetic pole, wherein the electromagnetic ring is arranged in the shell of the handle, the magnetic pole is fixedly arranged at a connecting part of the water jet tube, and a gap is provided between the magnetic pole and the electromagnetic ring, and the electromagnetic ring is configured to generate magnetism in turn along a circumferential direction by being energized, so as to attract the magnetic pole to move circumferentially and drive the distal end part of the water jet tube to swing circumferentially in two dimensions.

For example, the medical water jet scalpel device provided by at least one embodiment of the present disclosure further includes a supporting body, wherein the driving device includes a third motor, an eccentric structure and a second connector. The supporting body includes two fulcrum parts respectively arranged at two sides of the second connector; the third motor includes an output shaft along the central axis of the outer tube, the eccentric structure is sleeved on the output shaft of the third motor, one end of the second connector is connected with the eccentric structure, and the other end of the second connector is sleeved outside a connecting part of the water jet tube and is in clearance fit with the connecting part; the third motor is configured to drive the eccentric structure to revolve around the central axis of the outer tube through a rotation of the output shaft, so as to drive the second connector to move; and the second connector sequentially contacts with one of the two fulcrum parts to restrict the second connector from driving the connecting part to move circumferentially, so that the distal end part of the water jet tube swings circumferentially in two dimensions around the central axis of the outer tube.

At least one embodiment of the present disclosure also provides a medical water jet scalpel system, including a liquid supply unit, a pressure pump, and any medical water jet scalpel device described above. The liquid supply unit is configured to provide the medical liquid. The pressure pump is configured to apply a pressure to the medical liquid to input the medical liquid into the water jet tube.

Compared with the prior art, the beneficial effects of at least one embodiment of the present disclosure at least include: the medical water jet scalpel device or medical water jet scalpel system of the present disclosure automatically drives the water jet tube limited in the outer tube to move by means of a driving device, so that the water jet nozzle of the medical water jet scalpel device generates a rapid scanning movement, which is used to replace the rapid shaking action when a surgeon manually operates the medical water jet scalpel device, thus greatly reducing the operation difficulty, improving the surgical efficiency of selective separation by using water jet, and enhancing the effect of selective separation of tissues and the surgical accuracy.

### BRIEF DESCRIPTION OF DRAWINGS

In order to more clearly illustrate the technical solutions of the embodiments of the present disclosure or the prior art, the drawings necessary for description of the embodiments or the prior art will be briefly described in the following; it is obvious that the described drawings as below are only related to some embodiments of the present disclosure, from which other drawings can be obtained for those ordinary skilled in the art without creative labors.
Fig. 1 is a sectional view of a medical water jet scalpel device according to some embodiments of the present disclosure;
Fig. 2 is a partially enlarged view illustrating a part "A" of the medical water jet scalpel device of Fig. 1;
Fig. 3A is an exploded view of a movement path of a water jet nozzle of a medical water jet scalpel device according to some embodiments of the present disclosure;
Fig. 3B is an exploded view of a movement path of a water jet nozzle of a medical water jet scalpel device according to some other embodiments of the present disclosure;
Fig. 4A is a schematic diagram illustrating the principle of a one-dimensional reciprocating swing of a water jet nozzle of a medical water jet scalpel device according to some embodiments of the present disclosure;
Fig. 4B is a schematic diagram illustrating the principle of a two-dimensional circumferential swing of a water jet nozzle of a medical water jet scalpel device according to some embodiments of the present disclosure;
Fig. 4C is a schematic diagram illustrating the principle of a revolution of a water jet nozzle of a medical water jet scalpel device according to some embodiments of the present disclosure;
Figs. 5A-5C are schematic diagrams illustrating a water jet tube swinging inside an outer tube according to some embodiments of the present disclosure, and Figs. 5A and 5C are top views in the direction of arrow B in Fig. 4A or 4B, respectively;
Figs. 6A-6B are schematic diagrams illustrating a water jet tube rotating/revolving inside an outer tube according to some embodiments of the present disclosure, and Figs. 6A-6B are top views in the direction of arrow B in Fig. 4C, respectively;
Figs. 7-9 are schematic diagrams of driving devices for driving a water jet tube to rotate/revolve according to some embodiments of the present disclosure, respectively; wherein Fig. 7 is a sectional view of a built-in driving device including a hollow shaft motor, Fig. 8 is a sectional view of a built-in driving device including a micro motor, and Fig. 9 is a front view of an external driving device including a driving motor;
Fig. 10 is a sectional view of a driving device for driving a water jet tube to swing according to some embodiments of the present disclosure;
Figs. 11A-11B are schematic diagrams of a driving device for driving a water jet tube to rotate/revolve or swing according to some embodiments of the present disclosure; and
Fig. 12 is a schematic diagram illustrating components of a medical water jet scalpel system according to some embodiments of the present disclosure.

### DETAILED DESCRIPTION

Hereinafter, the technical solutions of the embodiments of the present disclosure will be described in a clearly and fully understandable way in connection with the drawings related to the embodiments of the present disclosure. Apparently, the described embodiments are just a part but not all of the embodiments of the present disclosure. Based on the described embodiments herein, all other embodiment(s) that can be obtained by those ordinary skilled in the art without any inventive work should be fallen within the scope of protection of the present disclosure.

Unless otherwise defined, all terms (including technical terms and scientific terms) used in the embodiments of the present disclosure have the same meanings as commonly understood by one of ordinary skilled in the art to which the present disclosure belongs. It should also be understood that terms such as those defined in a general dictionary should be interpreted to have meanings consistent with their meanings in the context of the relevant art, and are not to be interpreted in an idealized or overly formal sense, unless an embodiment of the present disclosure is explicitly defined so.

As used in the embodiments of the present disclosure, words such as "first", "second" and similarities do not indicate any order, quantity, or importance, but rather are used to distinguish between different components. Similar words such as "a", "an" or "the" do not indicate a limitation of quantity, but rather indicate the presence of at least one. Similarly, the use of the terms "comprising/including" or "comprise/include" and the like, is intended to mean that an element or article appearing before the word covers the listed element or article appearing after the word and its equivalents, but does not exclude other elements or articles. Similar terms such as "connect" or "connected" are not limited to physical or mechanical connections, but may include electrical or communicative connections, regardless of whether it's direct or indirect. Flowcharts are used in the embodiments of the present disclosure to illustrate steps of a method in accordance with the embodiments of the present disclosure. It should be understood that the preceding or following steps are not necessarily performed precisely in order. Rather, various steps may be carried out in reverse order or concurrently, unless the embodiments of the present disclosure are explicitly defined. Other operations can be added to these processes, or one or more steps are removed from these processes.

With the development of the field of medical surgery towards the direction of minimal invasion and refinement, there is still no method that can fully satisfy the surgical requirements for liver resection, effectively control the postoperative complications and improve the five-year survival rate after surgery among numerous liver resection methods. Therefore, innovations in existing medical technologies are imperative under current circumstances.

The liver is an organ with very complicated structure and abundant blood supply. The liver mainly includes two different types of tissues, one is soft liver parenchyma, and the other is tenacious lumen-like tissue. These two tissues are considerably different in their mechanical strengths. Therefore, under the impact of water jets of a medical water jet scalpel device, the liver parenchyma is split, broken and removed, and the lumen-like tissues remain intact and hence are preserved, so the medical water jet scalpel device can realize the selective separation function. Ideal methods of liver resection should have the following characteristics: high speed, no thermal damage, capability of vessel protection, realization of fine separation, minimal blood loss, easy operation under endoscope, avoidance or reduction of bile leakages and pleural effusions, and applicability for liver cirrhosis.

When the medical water jet scalpel device works, it can accomplish the cutting or removal of liver parenchyma as well as preservation and exposure of luminal-like tissues in a cutting zone with a certain width and length. In the cutting zone, due to the downward impact of water jets of the medical water jet scalpel device, part of the liver parenchyma is removed, for example, the depth is about 3 mm, and the originally buried lumen-like tissues are directly exposed to the surgeon's field of vision, which is convenient for the next operation of the surgeon. This method has no thermal damage to lumen-like tissues and parenchyma tissues. Performing the closing and disconnecting operations when the lumen-like tissues are clearly visible can also protect the lumen-like tissues and reduce the risk of surgery. In this process, there is very little bleeding, and it is not easy to cause bile leakage and effusion. It can be seen that compared with other methods of liver resection, medical water jet scalpel technology has great potential. However, the shortcomings of the medical water jet scalpel device, such as slow speed and difficult operation under endoscope, are problems that most surgeons can't accept and need to be solved urgently.

In this regard, at least one embodiment of the present disclosure provides a medical water jet scalpel device including an outer tube, a water jet tube, a water jet nozzle and a driving device. The water jet tube extends through the outer tube and is configured to input medical liquid. There is a gap between an outer wall of the water jet tube and an inner wall of the outer tube. The water jet nozzle is arranged at a distal end of the water jet tube and is configured to eject the medical liquid. The driving device is connected with at least part of the water jet tube, and is configured to drive a distal end part of the water jet tube to swing reciprocally or revolve around a central axis of the outer tube.

At least one embodiment of the present disclosure also provides a medical water jet scalpel system including the medical water jet scalpel device described above.

The medical water jet scalpel device of the above embodiment of the present disclosure automatically drives the water jet tube limited in the outer tube to move, such as swinging or rotating/revolving, by means of a driving device, so that the water jet nozzle of the medical water jet scalpel device generates a rapid scanning movement, such as a rapid lateral scanning movement, which is used to replace the rapid shaking action when a surgeon manually operates the medical water jet scalpel device, thus realizing the automatic scanning of the medical water jet scalpel device, greatly reducing the operation difficulty, improving the surgical efficiency of selective separation by using water jet, and improving the effect of selective separation of tissues and the surgical accuracy.

Hereinafter, embodiments of the present disclosure and examples thereof will be described in detail with reference to the accompanying drawings.

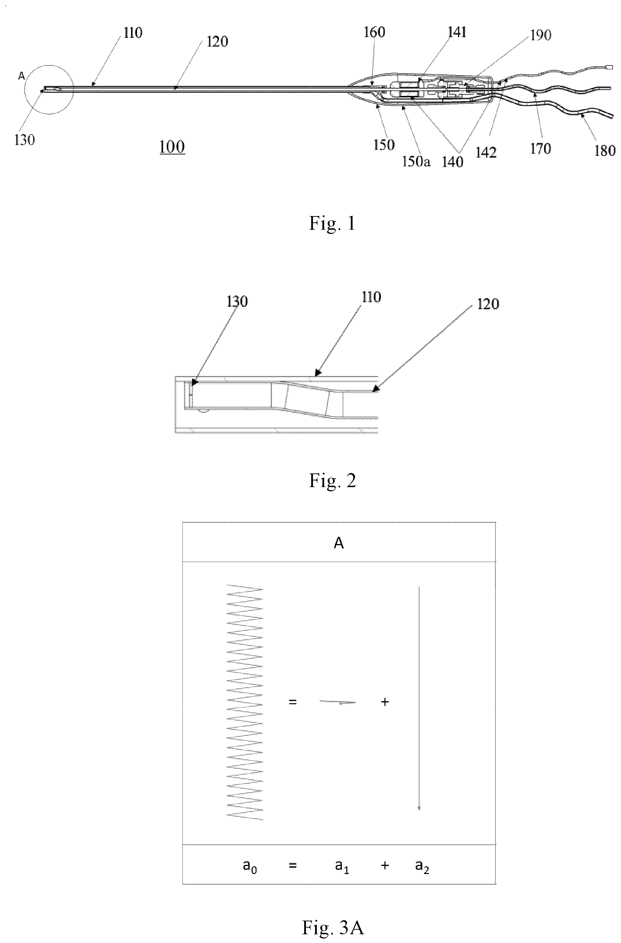

For convenience of expression, in the medical water jet scalpel device 100 in Fig. 1 according to an embodiment of the present disclosure, a proximal end of the outer tube 110 is closer to an operator, and a distal end of the outer tube 110 is away from the operator. At least one embodiment of the present disclosure regards the side close to the operator as a proximal end or a proximal end side, and regards the side away from the operator as a distal end or a distal end side. It should be noted that the distal end and the proximal end of the embodiment of the present disclosure are all relative positions, for example, they represent two opposite sides of some components themselves, or they represent two opposite sides in a certain direction; that is, in the embodiment of the present disclosure, the proximal end represents one side and the distal end represents the other side opposite to the proximal end.

For the sake of clarity and conciseness here, the following description mainly takes soft tissues as liver parenchyma tissues and elastic tissues as lumen-like tissues by way of example, but the embodiments of the present disclosure do not limit the types of soft tissues and elastic tissues that are applicable, that is, the present disclosure may also be applied to other biological tissues with similar needs and characteristics, such as parenchyma tissues and lumen-like tissues (such as blood vessels, bile ducts, ureters, bronchi, etc.) corresponding to other human organs (such as lung, kidney, spleen, etc.), which depends on actual situations and is not limited in the embodiment of the present disclosure.

Fig. 1 is a sectional view of a medical water jet scalpel device according to some embodiments of the present disclosure. Fig. 2 is a partially enlarged view illustrating part A of the medical water jet scalpel device of Fig. 1.

For example, as shown in Fig. 1 and Fig. 2, a medical water jet scalpel device 100 provided by at least one embodiment of the present disclosure includes an outer tube 110, a water jet tube 120, a water jet nozzle 130 and a driving device 140. The water jet tube 120 extends through the outer tube 110, and is configured to input pressurized medical liquid and allow for circulation of the medical liquid. There is a gap between an outer wall of the water jet tube 120 and an inner wall of the outer tube 110. The water jet tube 120 may also be referred to as an inner tube. The driving device 140 is connected with at least part of the water jet tube 120 and drives the water jet tube 120 to swing reciprocally or revolve around a central axis of the outer tube 110. The water jet nozzle 130 is arranged at a distal end of the water jet tube 120 and outputs the medical liquid. For example, one or more orifices in the water jet nozzle 130 generate a high-pressure jet of medical liquid, and the selective separation among liver parenchyma and, for example, blood vessels and bile ducts in vivo, is realized by using the jet.

In some examples, an inner diameter of the outer tube 110 is larger than an outer diameter of the water jet tube 120. For example, an inner diameter of the water jet tube 120 is about 1.5 mm to 5 mm, the outer diameter of the water jet tube 120 is about 2 mm to 6 mm, and a diameter of an orifice of the water jet nozzle 130 is about 0.05 mm to 0.15 mm. For example, a jet velocity of the medical liquid ejected from the water jet nozzle 130 is 50 m/s to 100 m/s.

In some examples, in order to complete the selective separation of the soft tissues and the elastic tissues in the whole cutting zone, the high-pressure jet ejected by the medical water jet scalpel device 100 needs to scan an area of the cutting zone, in turn, in the form of a Z-like path, which is convenient for a surgeon to perform the next operation of precise coagulation and occlusion of blood vessels. For example, a width of the cutting zone is 3 mm to 5 mm, and a length of the cutting zone is determined by the size of the biological tissues to be selectively separated here.

In some examples, the driving device 140 drives the water jet tube 120 to move so as to realize the movement of the water jet nozzle 130 fixedly arranged at the distal end of the water jet tube 120, and the movement of the water jet nozzle 130 is consistent with the movement of the distal end of the water jet tube 120. For example, a proximal end of the water jet tube 120 swings or rotates under the drive of the driving device 140, and the swing or rotation will be transmitted to the distal end of the water jet tube 120 and the water jet nozzle 130 fixed at the distal end, so that the water jet nozzle 130 swings or revolves correspondingly. Therefore, the swinging or revolving water jet nozzle 130 enables the high-pressure jet ejected from its orifice to realize the action of automatic scanning.

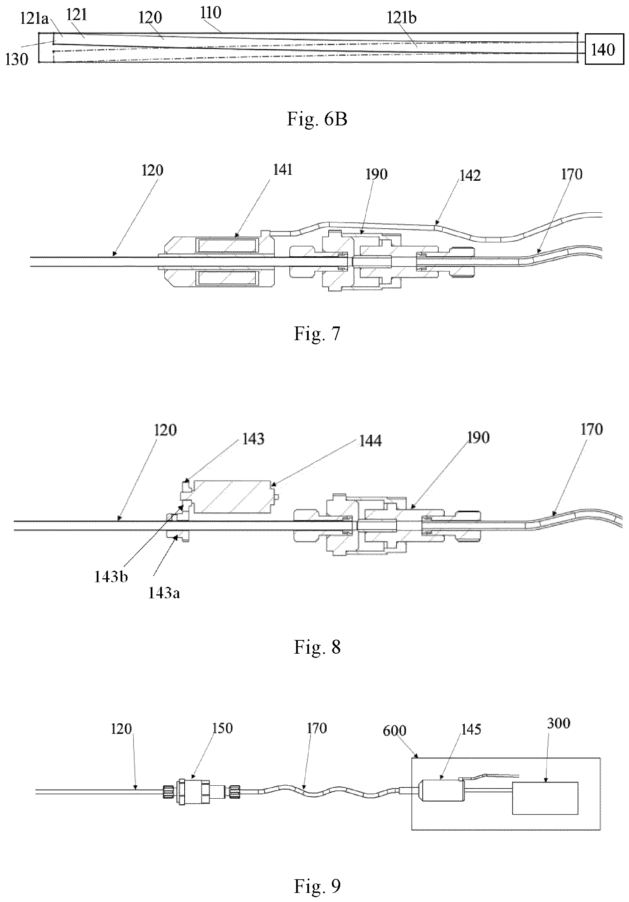

The driving device 140 of the medical water jet scalpel device 100 of Fig. 1 according to the embodiment of the present disclosure may be a hollow shaft motor, as shown in Fig. 7. However, the embodiment of the present disclosure is not limited to this. For example, it is also intended to show the following specific examples of the driving device of Figs. 8 to 10, that is, the examples of Figs. 8 to 10 may also be described in combination with the medical water jet scalpel device 100 of Fig. 1.

In some examples, the water jet nozzle 130 of the medical water jet scalpel device 100 moves slowly in a longitudinal direction while carrying out a rapid scanning movement in a transverse direction to form a Z-like path, so that the cutting zone is completely covered and the blood vessels in the cutting zone are fully exposed, which allows the liver parenchyma at the position where the high-pressure jet passes through to be cut off or removed, and allows the lumen-like tissues such as blood vessels, bile ducts and the like associated with the liver parenchyma to be preserved, thereby effectively realizing a selective separation. Therefore, in this process, the path of high-pressure jet can be decomposed into two different movements, such as a rapid reciprocating scanning movement in a transverse direction and a slow linear movement in a longitudinal direction.

As mentioned above, the medical water jet scalpel device 100 of the embodiment of the present disclosure automatically drives the water jet tube 120 in the outer tube 110 to swing or rotate/revolve by means of the driving device 140, so that the water jet nozzle 130 on the water jet tube 120 can generate, for example, the rapid scanning movement in the transverse direction instead of a high-frequency shaking movement when the surgeon manually operates the medical water jet scalpel device 100; in this way, the surgeon only needs to manually complete, for example, a slow linear movement in the longitudinal direction, thus considerably simplifying the operation and improving the surgical efficiency of the medical water jet scalpel device 100 for selective separation. Since the linear velocity and frequency of automatic transverse scanning are controllable, the optimal effect of selective separation can be obtained through experimental designs during the designing process, and the scanning width achieved by the automatic transverse scanning is consistent in a better way, the lateral cutting edge is smoother, and the damage to lumen-like tissues that needs to be preserved is minimal, thus greatly improving the surgical effect and accuracy. Automatic scanning can also avoid the damage to the lumen-like tissues caused by the jet of the water jet nozzle 130 of the medical water jet scalpel device 100 staying in a certain position for too long, thus further reducing the bleeding amount and the probability of related accidents during the surgery operation using the medical water jet scalpel device. Furthermore, since it is not necessary to swing manually when using the medical water jet scalpel device, the outer tube 110 has no transverse movement relative to the liver in a slit of a cutting edge, thus eliminating the risk that the blood vessel will be pulled and broken after the outer tube 110 collides with a side wall of the cutting edge of the tissue. Therefore, the embodiment of the present disclosure solves the problem that an existing endoscopic-type medical water jet scalpel device is difficult to exert its advantages for selective separation of tissues, and satisfies increasingly growing, critical clinical requirements of the endoscopic-type medical water jet scalpel device.

In some examples, a proximal end part of the water jet tube 120 includes a connecting part 122 fixedly connected with at least part of the driving device 140, and the distal end part of the water jet tube 120 and the connecting part 122 of the water jet tube 120 are not coaxial with each other, so that the driving device 140 drives the proximal end part of the water jet tube 120 to rotate around the central axis of the water jet tube 120 and enables the distal end part of the water jet tube 120 to revolve around the central axis of the outer tube 110.

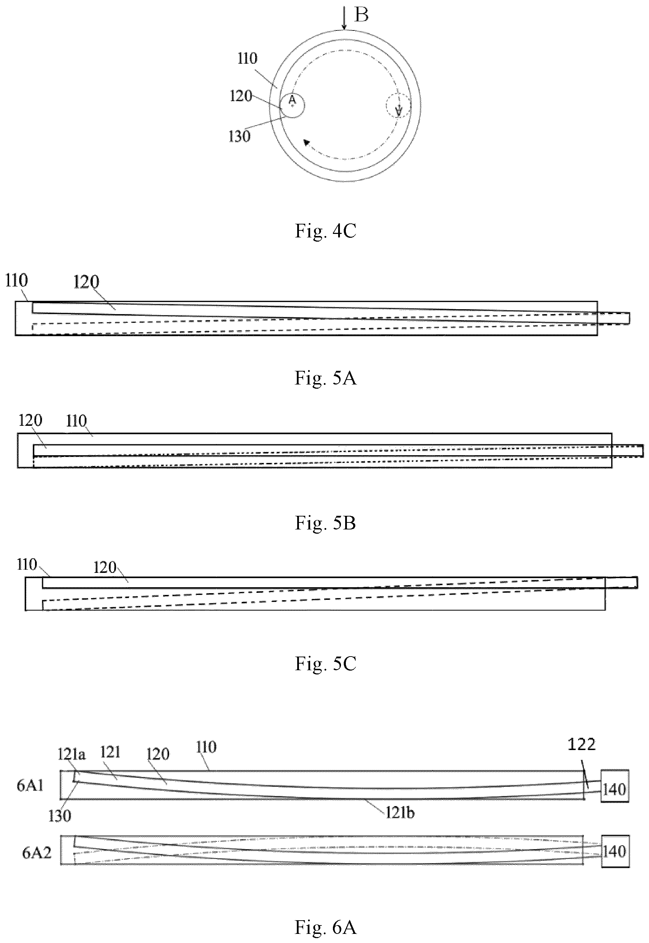

For example, at least part of the driving device 140 is fixedly connected with the proximal end part of the water jet tube 120, and the water jet tube 120 is integrally bent (see Figs. 6A and 6B described below for details), so that the driving device 400 drives the proximal end part of the water jet tube 120 to rotate around the central axis of the water jet tube 120 and enables the distal end part of the water jet tube 120 to revolve around the central axis of the outer tube 110.

For another example, at least part of the driving device 140 is fixedly connected with the proximal end part of the water jet tube 120, and the distal end part of the water jet tube 120 is a bending part and hence has at least two segments which are not coaxial with each other (for example, it's partially bent as shown in Fig. 2), so that the driving device 140 drives the proximal end part of the water jet tube 120 to rotate around the central axis of the water jet tube 120 and enables the distal end part of the water jet tube 120 to revolve around the central axis of the outer tube 110.

For example, as shown in Fig. 2, the water jet tube 120 makes a relative movement in the outer tube 110, and the distal end part of the water jet tube 120 abuts against an inner wall of one side of the outer tube 110, so that when the water jet tube 120 is driven, by the driving device 140, to swing or rotate/revolve, the scanning width realized by the high-pressure jet of the water jet nozzle 130 is relatively larger, which satisfies the requirements for the width of a cutting zone in clinical applications. For example, in the example of Fig. 2, the distal end part of the water jet tube 120 is bent, or in the example of Figs. 6A-6B, the water jet tube 120 is integrally bent, so that the water jet nozzle 130 of the water jet tube 120 abuts against the inner wall of the outer tube 110. This is merely exemplary and is not a limitation of the present disclosure.

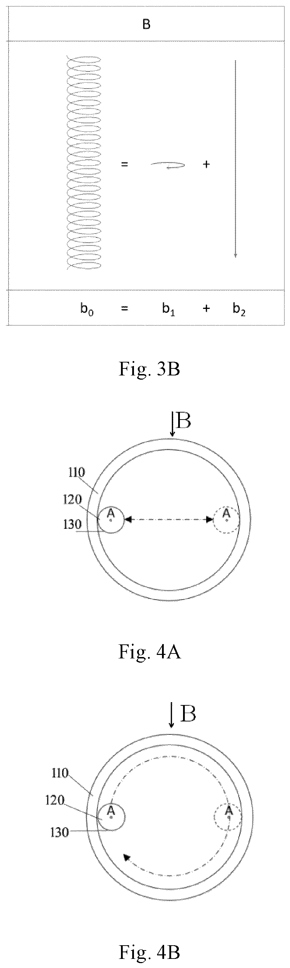

Fig. 3A is an exploded view of a movement path of the water jet nozzle of the medical water jet scalpel device according to some embodiments of the present disclosure. Fig. 3B is an exploded view of a movement path of the water jet nozzle of the medical water jet scalpel device according to some other embodiments of the present disclosure.

For example, as shown in Fig. 3A, when the medical water jet scalpel device 100 is working, the water jet nozzle 130 can adopt a Z-like a₀ path to realize the selective separation of tissues, and the a₀ path may be decomposed into a reciprocating movement a₁ and a linear movement a₂.

In some examples, the reciprocating movement a₁ is a reciprocating linear movement in a transverse direction, and the reciprocating movement a₁ may be driven by the driving device 140. The linear movement a₂ is a linear movement in a longitudinal direction, and the linear movement a₂ may be manually completed by a surgeon. Therefore, the medical water jet scalpel device 100 of the embodiment of the present disclosure can automatically realize the reciprocating movement a₁. Compared with the facts found by the inventors that some medical water jet scalpel devices completely rely on the surgeons to manually realize the a₀ path of the water jet nozzle 130, the embodiment of the present disclosure can greatly reduce the operation difficulty, improve the operation efficiency, improve the usability of the medical water jet scalpel device 100, solve the problems of poor repeatability and time-consuming and laborious operation caused by surgeons' rapid manual shaking to realize the reciprocating movement in the transverse direction, and also solve the problem that the realization of selective separation function of the endoscopic water jet is seriously affected because the medical water jet scalpel device under the endoscope cannot realize accurate and rapid transverse scanning movement.

For example, as shown in Fig. 3B, when the medical water jet scalpel device 100 is working, the water jet nozzle 130 can adopt a Z-like b₀ path to realize the selective separation of tissues, and the b₀ path may be decomposed into a reciprocating movement b₁ and a linear movement b₂.

In some examples, the reciprocating movement b₁ is a circumferential movement in a transverse direction, and the reciprocating movement b₁ may be driven by the driving device 140. The linear movement b₂ is a linear movement in a longitudinal direction, and the linear movement b₂ may be manually completed by a surgeon. Therefore, the medical water jet scalpel device 100 of the embodiment of the present disclosure can automatically realize the reciprocating movement b₁. Compared with the fact found by the inventors that some medical water jet scalpel devices completely rely on the surgeons to manually realize the b₀ path of the water jet nozzle 130, the embodiment of the present disclosure can greatly reduce the operation difficulty, improve the operation efficiency, improve the usability of the medical water jet scalpel device100, solve the problems of poor repeatability and time-consuming and laborious operation caused by surgeons' rapid manual shaking to realize the transverse reciprocating movement, and also solve the problem that the realization of selective separation function of the endoscopic water jet is seriously affected because the medical water jet scalpel device under the endoscope cannot realize accurate and rapid transverse scanning movement.

In some examples, the longitudinal linear movement may travel along a cutting edge with a safe distance from the tissues to be selectively separated, which may be planned and manually controlled by the surgeon. The transverse reciprocating movement may also be referred to as transverse scanning movement, with the purpose of allowing the cutting zone to have enough width to facilitate the subsequent treatment of blood vessels. The width of the cutting zone is generated by the transverse scanning movement of the high-pressure jet of the water jet nozzle of the medical water jet scalpel device, that is, the width of the cutting zone is equal to the transverse distance of the scanning movement. For example, the above-mentioned transverse and longitudinal directions are relative positions, which represent two directions perpendicular to each other, depending on the position of the tissue to be selectively separated, and will not be repeated here.

In some examples, a speed of the longitudinal linear movement is about 1 mm/s, and a speed of the transverse reciprocating movement is about 40 mm/s. The speed of the transverse reciprocating movement is about 40 mm/s, which can replace the surgeon's high-frequency wrist-shaking operation; for example, the frequency of the surgeon's manual shaking is about 5 Hz.

Fig. 4A is a schematic diagram illustrating the principle of a one-dimensional reciprocating swing of the water jet nozzle of the medical water jet scalpel device according to some embodiments of the present disclosure. Fig. 4B is a schematic diagram illustrating the principle of a two-dimensional circumferential swing of the water jet nozzle of the medical water jet scalpel device according to some embodiments of the present disclosure. Fig. 4C is a schematic diagram illustrating the principle of a revolution of the water jet nozzle of the water jet scalpel device according to some embodiments of the present disclosure.

For example, in Figs. 4A, 4B and 4C, an outer solid-line circle refers to an end face of the outer tube 110, a small solid-line circle at the left side in the solid-line circle refers to the water jet nozzle 130 at the distal end of the water jet tube 120, dotted lines and arrows indicate a scanning path and a moving direction of the water jet nozzle 130, and a small dotted-line circle at the right side in the solid-line circle indicates a possible position of the water jet nozzle 130 after the movement. "A" is used to indicate the direction of the water jet nozzle 130 itself, and the inverted "A" indicates that the water jet nozzle 130 has been rotated around the central axis of the water jet tube 120 by 180 degrees at the corresponding position.

In some examples, the proximal end part of the water jet tube 120 is fixed relative to the outer tube 110, and the driving device 140 is configured to drive the distal end part of the water jet tube 120 to swing reciprocally in one dimension along a radial direction of the water jet tube 120 relative to the outer tube 110, or to swing circumferentially in two dimensions around the central axis of the outer tube 110. For example, the driving device 140 drives the proximal end part of the water jet tube 120 to perform one-dimensional reciprocating swing or two-dimensional circumferential swing to realize the one-dimensional reciprocating swing or the two-dimensional circumferential swing of the distal end part of the water jet tube 120. In this way, the scanning width required by the water jet nozzle 130 is achieved by driving the water jet tube 120 limited in the outer tube 110 to swing.

In some examples, the water jet nozzle 130 fixed on the water jet tube 120 swings reciprocally in one dimension along the radial direction of the water jet tube 120, thereby realizing the reciprocating movement a₁. For example, the swing of the water jet nozzle 130 along one radial direction of the water jet tube 120 is recorded as a one-dimensional swing along the transverse direction. For example, the water jet nozzle 130 performs a reciprocating linear movement along the dotted line and arrow direction in Fig. 4A, and the direction of the water jet nozzle 130 itself is not deflected.

In some examples, the water jet nozzle 130 on the water jet tube 120 swings circumferentially in two dimensions around the central axis of the outer tube 110 along the radial direction of the water jet tube 120, thereby realizing the reciprocating movement b₁. For example, the water jet nozzle 130 moves circumferentially around the central axis of the outer tube 110 along the dotted line and arrow direction in Fig. 4B, and the direction of the water jet nozzle 130 itself is not deflected.

As can be seen from Figs. 4A and 4B, when the distal end part of the water jet tube 120 swings, the direction of the water jet nozzle 130 itself has no deflection regardless of the path, that is, the water jet nozzle 130 does not rotate around its own axis.

In some examples, when the water jet tube 120 rotates around the central axis of the water jet tube 120 (that is, the water jet tube rotates around its own axis), the direction of the water jet nozzle 130 itself has been deflected, as shown in Fig. 4C, and the distal end part of the water jet tube 120 revolves around the central axis of the outer tube 110, thereby realizing the reciprocating movement b₁.

According to Fig. 4C, the proximal end part of the water jet tube 120 rotates around the central axis of the water jet tube 120 and the distal end part of the water jet tube 120 moves around the central axis of the outer tube 110 (that is, the distal end part of the water jet tube 120 revolves around the central axis of the outer tube 110), and a revolution period is equal to a rotation period. When the water jet tube 120 is driven to rotate and revolve, the water jet nozzle 130 moves correspondingly with the movement of the distal end part of the water jet tube 120.

In some embodiments of the present disclosure, "rotation" (or "rotate") and "revolution" (or "revolve") both refer to moving around an axis (for example, the axis of revolution is the central axis of the outer tube and the axis of rotation is the central axis of the water jet tube), and "revolution" (or "revolve") is a movement concept relative to "rotation" (or "rotate"). For example, the revolving axis for "revolution" and the rotating axis for "rotation" may be different (for example, the central axis of the water jet tube 120 and the central axis of the outer tube 110 are different), and the radial sizes and trajectories of revolution and rotation around the axes are different.

In some embodiments of the present disclosure, the two-dimensional circumferential swing itself is a movement mode in which a component, as a whole, passes through a circle around an axis, and each point of the component itself is in translation movement rather than rotating/revolving movement. The two-dimensional circumferential swing is also a term determined to be distinguished from the revolution in the above solution with rotation. It should be noted that the meanings and functions of elements or objects in the embodiments of the present disclosure are not limited by their names, and cannot be interpreted in an idealized or extremely formal sense. The specific meanings of revolution, rotation and two-dimensional circumferential swing of the present disclosure need to be understood by those skilled in the art according to the full text of the present disclosure, which does not limit the scope of protection of the present disclosure.

Figs. 5A-5C are schematic diagrams illustrating the water jet tube swinging inside the outer tube according to some embodiments of the present disclosure, and Figs. 5A and 5C are top views in the direction of arrow B in Fig. 4A or 4B, respectively.

In some examples, the central axis of the water jet tube 120 and the central axis of the outer tube 110 may be coaxial with each other, and may also be arranged in parallel or arranged at an angle.

For example, as shown in Fig. 5A, before the water jet tube 120 is driven, the central axis of the water jet tube 120 is not parallel to the central axis of the outer tube 110, and the central axis of the water jet tube 120 is arranged at an angle with relative to the central axis of the outer tube 110; and the distal end part of the water jet tube 120 is arranged to abut against an inner wall of one side of the outer tube 110 in a radial direction, and the proximal end part of the water jet tube 120 is arranged with gaps relative to both sides of the outer tube 110 in the radial direction.

For example, in the example of Fig. 5A, the water jet tube 120 has a one-dimensional or two-dimensional swing space in the outer tube 110. The solid line inside the outer tube 110 in Fig. 5A indicates an initial position of the water jet tube 120, and the dotted line inside the outer tube 110 in Fig. 5A indicates a possible position of the water jet tube 120 after swinging. In this way, the scanning width realized by the high-pressure jet of the water jet nozzle 130 fixed at the distal end of the water jet tube 120 is relatively larger and satisfies the requirements for a width of the cutting zone in clinical applications.

For example, as shown in Fig. 5B, before the water jet tube 120 is driven, the central axis of the water jet tube 120 is coaxial with the central axis of the outer tube 110, and both the distal end part and the proximal end part of the water jet tube 120 are arranged with gaps relative to both sides of the outer tube 110 in the radial direction.

For example, in the example of Fig. 5B, the water jet tube 120 also has a one-dimensional or two-dimensional swing space in the outer tube 110. The solid line inside the outer tube 110 in Fig. 5B indicates an initial position of the water jet tube 120, and the dotted line inside the outer tube 110 in Fig. 5B indicates a possible position of the water jet tube 120 after swinging. Thus, the scanning width realized by the high-pressure jet of the water jet nozzle 130 fixed at the distal end of the water jet tube 120 in the example of Fig. 5B is smaller than the scanning width realized by the high-pressure jet of the water jet nozzle 130 fixed at the distal end of the water jet tube 120 in the example of Fig. 5A. Therefore, the example of Fig. 5B can satisfy the requirements for a width of the cutting zone in some of the clinical applications.

For example, as shown in Fig. 5C, before the water jet tube 120 is driven, the central axis of the water jet tube 120 and the central axis of the outer tube 110 are parallel to each other and are not coaxial with each other, and both the distal end part and the proximal end part of the water jet tube 120 are arranged to abut against the inner wall of a same side of the outer tube 110 in the radial direction.

For example, in the example of Fig. 5C, the water jet tube 120 has a one-dimensional or two-dimensional swing space in the outer tube 110. The solid line inside the outer tube 110 in Fig. 5C indicates an initial position of the water jet tube 120, and the dotted line inside the outer tube 110 in Fig. 5C indicates a possible position of the water jet tube 120 after swinging. In this way, the scanning width realized by the high-pressure jet of the water jet nozzle 130 fixed at the distal end of the water jet tube 120 is relatively larger and satisfies the requirements for a width of the cutting zone in clinical applications.

Based on the foregoing, in the embodiment of the present disclosure, at least part of the water jet tube 120 is arranged with a gap relative to one side of the outer tube 110 in the radial direction, which can provide at least part of a space for the swinging movement of the water jet tube 120 in the outer tube 110. The size of the gap between the water jet tube 120 and the outer tube 110 determines the scanning width realized by the high-pressure jet of the water jet nozzle 130 fixed at the distal end of the water jet tube 120.

For example, for the example of Figs. 5A, 5B and 5C, the driving device 140 may be arranged at the proximal end of the water jet tube 120. By driving the proximal end part of the water jet tube 120 to swing, the distal end part of the water jet tube 120 may be driven to swing and then the water jet nozzle 130 is driven to swing. This is only exemplary, and is not a limitation of the present disclosure. For example, the swing of the distal end part of the water jet tube 120 can be directly realized by directly applying a driving force to the distal end part of the water jet tube 120, which will not be described in detail here.

Figs. 6A-6B are schematic diagrams illustrating the water jet tube moving (e.g., revolving and rotating) inside the outer tube provided by some embodiments of the present disclosure, and Figs. 6A-6B are top views in the direction of arrow B in Fig. 4C, respectively.

For example, as shown in Figs. 6A and 6B, the proximal end part of the water jet tube 120 (for example, the connecting part of the water jet tube 120 is the proximal end of the water jet tube 120 close to the driving device 140) is fixedly connected with at least part of the driving device 140, and the driving device 140 drives the proximal end part of the water jet tube 120 to rotate around the central axis of the water jet tube 120. The water jet tube 120 is integrally bent, and the water jet nozzle 130 is provided at the distal end of the water jet tube. The driving device 140 drives the proximal end part of the water jet tube 120 to rotate around the central axis of the water jet tube 120, thus realizing the revolution of the distal end part of the water jet tube 120 around the central axis of the outer tube 110. Therefore, the embodiment of the present disclosure achieves the scanning width realized by the high-pressure jet of the water jet nozzle 130 by driving the water jet tube 120 to rotate/revolve, with wider application range and higher practical application value.

For example, as shown in Fig. 6A, the water jet tube 120 is integrally bent. When a distal end part 121a of the water jet tube 120 abuts against an inner wall of a first side of the outer tube 110 along a radial direction, a proximal end part 121b of the water jet tube 120 abuts against an inner wall of the other side of the outer tube 110 opposite to the first side along the radial direction. Therefore, the water jet tube 120 does not have the freedom to swing in the outer tube 110, and the water jet tube 120 is confined in the outer tube 110 and cannot swing. As shown in Fig. 6A, the solid line inside the outer tube 110 indicates an initial position of the water jet tube 120, and the water jet tube 120 cannot realize the transverse scanning movement of the water jet nozzle 130 by swinging.

For example, as shown in Fig. 6A, the water jet tube 120 still has freedom(s) of rotating/revolving in the outer tube 110, and the proximal end part of the water jet tube 120 is driven to rotate by the driving device 140, so that the water jet tube 120 can rotate around its own axis. The water jet tube 120 has a curved part 121, so that when the proximal end part of the water jet tube 120 rotates around the central axis of the water jet tube, the curved part 121 of the water jet tube 120 is shaken, and the movement trajectory forms a ring around the central axis of the outer tube 110. Therefore, the distal end part 121a of the water jet tube 120 can realize a revolution around the central axis of the outer tube 110. For example, as shown in Fig. 6A, the dotted line inside the outer tube 110 indicates a possible position of the water jet tube 120 after rotating/revolving.

For example, as shown in Fig. 6B, the distal end part 121a of the water jet tube 120 abuts against an inner wall of a first side of the outer tube 110 along a radial direction, and there is a gap between the proximal end part 121b of the curved part 121 and an inner wall of the other side of the outer tube 110 opposite to the first side along the radial direction.

For example, as shown in Fig. 6B, the water jet tube 120 has freedom(s) of rotating/revolving in the outer tube 110, and the proximal end part of the water jet tube 120 is driven to rotate by the driving device 140, so that the water jet tube 120 can rotate around its own axis. The water jet tube 120 has a curved part 121, so that when the proximal end part of the water jet tube 120 rotates around the central axis of the water jet tube 120, the curved part 121 of the water jet tube 120 is shaken, and the movement trajectory forms a ring around the central axis of the outer tube 110. Therefore, the distal end part 121a of the water jet tube 120 can realize a revolution around the central axis of the outer tube 110. For example, the dotted line inside the outer tube 110 in Fig. 6B indicates a possible position of the water jet tube 120 after rotating/revolving.

For example, as shown in Figs. 6A and 6B, the bending degree of the water jet tube 120 shown in Fig. 6A is greater than the bending degree of the water jet tube 120 shown in Fig. 6B.

Thus, the embodiment of the present disclosure realizes the revolution of the distal end part of the water jet tube 120 by driving the proximal end part of the water jet tube 120 to rotate around the central axis of the water jet tube 120, thereby realizing the scanning width required by the water jet nozzle 130. Since the distal end part of the water jet tube 120 abuts against an inner wall of one side of the outer tube 110 along a radial direction, in this case, the scanning width realized by the high-pressure jet of the water jet nozzle 130 fixed at the distal end of the water jet tube 120 is relatively larger and satisfies the requirements for a width of the cutting zone in clinical applications.

It should be noted that in the example of Fig. 6B, the water jet tube 120 still has a certain swing space in the outer tube 110. Therefore, in some cases where there are lower requirements for the width of the cutting zone, the scanning width required by the water jet nozzle can be achieved by swinging in the example of Fig. 6B. However, in some cases where there are higher requirements for the width of the cutting zone, the expected effect may not be achieved because the swing amplitude of the water jet tube in the example of Fig. 6B is restricted to a certain extent, so it is better to achieve the scanning width required by the water jet nozzle by rotating/revolving.

In some examples, the distal end part of the water jet tube 120 is configured as a bending part, so that the distal end part has at least two segments which are not coaxial with each other. For example, a distal end of the distal end part itself (i.e., a left end of the distal end part) and a proximal end of the distal end part itself (i.e., a right end of the distal end part) are at least not coaxial with each other by bending the distal end part of the water jet tube 120 in the example of Fig. 2. For example, the bending part in the example of Fig. 2 has three segments which are not coaxial with each other. This is merely exemplary and is not a limitation of the present disclosure.

It should be noted that, in some examples, the distal end part of the water jet nozzle 130 refers to a part of the water jet nozzle 130 that is at a side away from the operator and has a certain length; the water jet nozzle 130 is fixed at the distal end of the water jet tube 120, which means that the water jet nozzle 130 is fixed at a distal end side of the distal end part of the water jet tube 120, and the water jet nozzle 130 is fixed at a distal end which is not coaxial with the connecting part of the water jet tube 120. In this way, the water jet nozzle 130 can generate a rotation and a revolution. For example, in the example of Fig. 2, the distal end of the water jet tube 120 abuts against an inner wall of a first side of the outer tube 110 along the radial direction, and the proximal end part of the water jet tube 120 may be arranged with a gap relative to an inner wall of the other side of the outer tube 110 opposite to the first side along the radial direction.

It should also be noted that, in some examples, the above-mentioned bending part or bending may be linear bending and may also be curved bending, and it may be smooth bending or non-smooth bending, and the implementation of the present disclosure is not limited to this, as long as the axes of different segments are not in a straight line, which is not repeated here.

In some examples, the position and shape of the water jet tube 120 in the outer tube 110 illustrated in Fig. 6A are generated for reasons as below: an ideal state of processing the outer tube 110 and the water jet tube 120 is a straight tube which has good cylindricity. In this case, the water jet tube 120 may be enabled to swing in the outer tube 110, as described above, which is not repeated here. Moreover, in some specific cases, for example, for an endoscopic device, the outer tube 110 and the water jet tube 120 have longer lengths, for example, the length of the water jet tube 120 needs to reach about 400 mm, and the outer tube 110 with a larger diameter can basically maintain a good cylindricity. However, a thin-walled water jet tube 120 with a smaller diameter is prone to be curved/bent to a certain extent at a length of 400 mm, which is similar to the possible situation shown in Fig. 6A or 6B.

Therefore, compared with the method of driving the swing of the water jet tube 120 to achieve the required scanning width of the water jet nozzle 130, in the method of driving the rotation/revolution of the water jet tube 120 to achieve the required scanning width of the water jet nozzle 130 in the embodiment of the present disclosure, the achievable scanning width is not affected by the bending of the water jet tube 120, which allows the water jet tube 120 to have a certain degree of bending. This method has lower processing requirements for the water jet tube 120 and the outer tube 110, with wider application range and higher practical application value.

It should be noted that the embodiments of the present disclosure shown in Figs. 5A, 5B and 5C can also achieve the scanning width required by the water jet nozzle 130 by driving the water jet tube 120 to rotate/revolve, as long as the distal end side of the water jet tube 120 partially has a curved part. For details, reference can be made to the examples of Figs. 6A and 6B, which will not be repeated here.

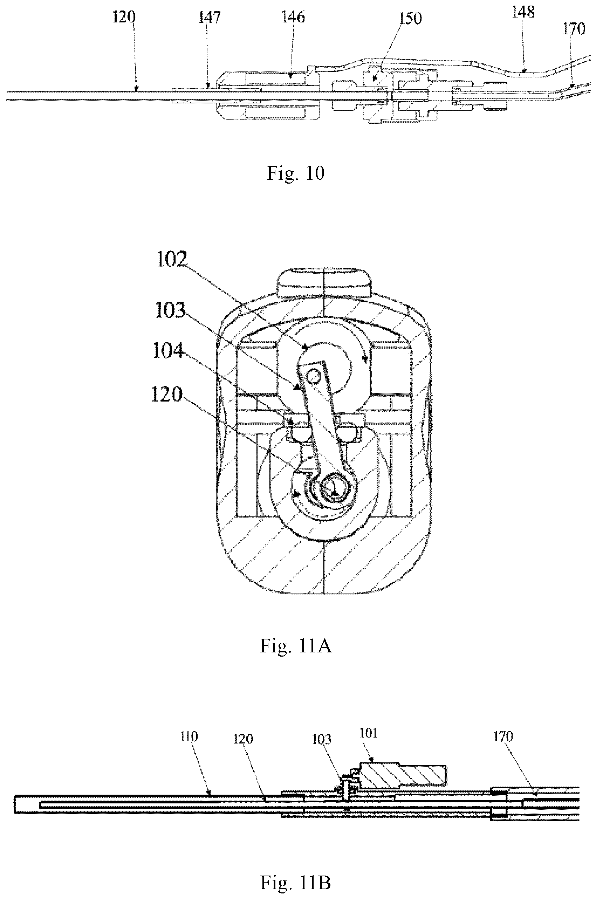

Figs. 7-9 are schematic diagrams of a driving device for driving the water jet tube to rotate/revolve according to some embodiments of the present disclosure, respectively. Fig. 7 is a sectional view of a built-in driving device including a hollow shaft motor, Fig. 8 is a sectional view of a built-in driving device including a micro motor, and Fig. 9 is a front view of an external driving device including a driving motor. Fig. 10 is a sectional view of a driving device for driving the water jet tube to swing according to some embodiments of the present disclosure.

For example, as shown in Figs. 1 and 7-10, the medical water jet scalpel device 100 further includes a supporting body 150 (such as the following handle 150a), which is sleeved outside the proximal end of the outer tube 110 and fixedly connected with the proximal end of the outer tube 110. In this way, it is convenient to operate the medical water jet scalpel device 100, and it is also beneficial to installing components of the medical water jet scalpel device 100.

In some examples, the supporting body 150 includes a handle 150a, and the handle 150a is fixedly connected with the driving device 140. The handle 150a is configured to be driven by an external driving source to move in a direction of the central axis of the outer tube 110, so that the distal end part of the water jet tube 120 moves in the direction of the central axis of the outer tube 110, thereby forming a Z-like scanning path. For example, the external driving source may be manually operated by a surgeon to complete the longitudinal linear movement, which is only exemplary and not limited in the present disclosure. For example, the handle 150a may also be driven by an automatic driver, which will not be described in detail here. In this way, the water jet nozzle of the medical water jet scalpel device of the embodiment of the present disclosure can form a Z-like path, so as to fully cover the cutting zone, thereby fully exposing the blood vessels in the cutting zone, cutting or removing the liver parenchyma at the position where the high-pressure jet passes, preserving the lumen-like tissues such as blood vessels and bile ducts associated with the liver parenchyma, and effectively completing selective separation.

In some examples, the driving device 140 may be realized by a motor-type driving device driven by electric energy, and the driving device 140 may also be driven by other forms of power sources. The driving device 140 may be arranged in a shell of the handle 150a, and may also be arranged outside a shell of the handle 150a. For example, the driving device 140 is arranged outside the shell of the handle 150a and arranged in a host system (see the host system 600 described below).

In some examples, the driving device 140 includes a driving motor, and the handle 150a includes a shell, and the driving motor is arranged inside or outside the shell of the handle 150a. The driving motor is connected with the connecting part 122 of the water jet tube 120 to drive the proximal part of the water jet tube 120 to rotate around the central axis of the water jet tube 120. Therefore, the embodiment of the present disclosure uses electric energy to drive the movement of the water jet tube, which is convenient for operation, simple in assembly and strong in controllability.

For example, as shown in Fig. 7, the driving motor of the driving device 140 includes a first motor 141, and the first motor 141 is arranged in the shell of the handle 150a. The driving device 140 further includes a driver connecting line 142, and the first motor 141 is configured to obtain electric energy and a control signal through the driver connecting line 142. For example, the first motor 141 is connected to at least part of a host system (see the host system 600 described below) through the driver connecting line 142 to obtain the electric energy and the control signal. The first motor 141 is sleeved outside the connecting part 122 of the water jet tube 120 and is coaxially fixed with the water jet tube 120 to drive the proximal end part 121b of the water jet tube 120 to rotate around the central axis of the water jet tube 120.

For example, in the example of Fig. 7, the first motor 141 is a hollow shaft motor, and an inner hole of the hollow shaft motor is in tight fit with the outer diameter of the water jet tube 120, so that the hollow shaft motor and the water jet tube 120 are coaxially fixed with each other, with simple layout and reliable structure. For example, when the first motor 141 receives the control signal and starts to rotate, it drives the proximal end of the water jet tube 120 to rotate, so that the high-pressure jet of the water jet nozzle 130 at the distal end of the water jet tube 120 generates a reciprocating scanning movement.

For example, as shown in Fig. 8, the driving motor of the driving device 140 includes a second motor 144, and the driving device 140 further includes a first connector 143, and the second motor 144 and the first connector 143 are arranged in the shell of the handle 150a. The first connector 143 is fixedly connected with the proximal end of the water jet tube 120, and the second motor 144 is connected with the first connector 143 and configured to drive the first connector 143 to rotate so as to drive the proximal end part of the water jet tube 120 to rotate around the central axis of the water jet tube 120.

For example, the second motor 144 is a micro motor, which may refer to a motor with a diameter less than 160 mm or a rated power less than 750 mW. Therefore, in the embodiment of the present disclosure, the micro motor is used to drive the water jet tube 120 to rotate/revolve, and the micro motor has a smaller volume and weight, which makes it easier for the operator to control and less likely for the operator to feel tired when using the medical water jet scalpel device.

In some examples, the first connector 143 includes at least one of the following: a gear, a belt pulley, and a connecting rod. In this way, it can be connected with the proximal end of the water jet tube 120, and the water jet tube 120 can be driven to rotate/revolve by the micro motor, so that the jet of the water jet nozzle 130 at the distal end of the water jet tube 120 can generate the reciprocating scanning movement. This is only an example, and is not a limitation of the present disclosure. It only needs to be in tight fit with the water jet tube 120 and connected with the micro motor, which will not be described in detail here.

For example, as shown in Fig. 8, the first connector 143 includes a first gear 143a and a second gear 143b, and the first gear 143a is meshed with the second gear 143b. The first gear 143a is fixedly connected with the connecting part 122 of the water jet tube, and the second gear 143b is connected with the second motor 144.

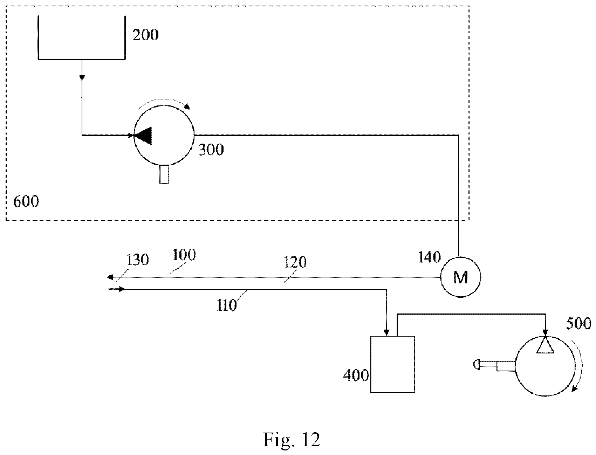

For example, as shown in Figs. 1 and 7-9, the medical water jet scalpel device 100 further includes a liquid supply connecting pipeline 170, for example, the liquid supply connecting pipeline 170 is a high-pressure hose. One end of the liquid supply connecting pipeline 170 is connected with the proximal end of the water jet tube 120, and the other end of the liquid supply connecting pipeline 170 is configured to be connected with an output end of a pressure pump (see the pressure pump 300 shown in Fig. 12 and described below). Thus, by means of the pressure pump, the medical liquid passes through the liquid supply connecting pipeline 170 and flows through the water jet tube 120 until it is ejected from the orifice of the water jet nozzle 130.

For example, as shown in Fig. 9, the driving motor 145 of the driving device 140 is arranged outside the shell of the handle 150a, that is, the driving motor 145 may also be referred to as an external motor. The driving motor 145 is fixedly connected with the liquid supply connecting pipeline 170 and configured to drive the liquid supply connecting pipeline 170 to rotate so as to drive the proximal end part of the water jet tube 120 to rotate around the central axis of the water jet tube 120. Therefore, the embodiment of the present disclosure drives the water jet tube 120 to rotate/revolve by adopting an external motor, so that the handle 150a of the medical water jet scalpel device 100 is small in size, light in weight and good in maneuverability, and the transmission and vibration inside the handle 150a are also reduced. Moreover, the handle assembly is not electrified, and the external motor may be arranged in a host system (see the host system 600 described below) for repeated use, which can reduce the cost of consumables. For example, a connection line from a host system (see the host system 600 described below) to the handle 150a may be omitted.

In some examples, the liquid supply connecting pipeline 170 adopts a metal braided pipe, and the axial torsional rigidity of the liquid supply connecting pipeline 170 may be used for transmitting a torque. In this way, the movement of the proximal end of the water jet tube 120 can be realized by the driving motor 145 driving the liquid supply connecting pipeline 170, so that the jet of the water jet nozzle 130 at the distal end of the water jet tube 120 can generate the reciprocating scanning movement.

For example, as shown in Figs. 1 and 7-9, the liquid supply connecting pipeline 170 is connected with the proximal end of the water jet tube 120 through a high-pressure adapter 190.

For example, as shown in Fig. 10, the driving device 140 includes an electromagnetic ring 146 and a magnetic pole 147. The electromagnetic ring 146 is arranged in the shell of the handle 150a, and the magnetic pole 147 is fixedly arranged at the proximal end of the water jet tube 120 and has a gap with relative to the electromagnetic ring 146. When the electromagnetic ring 146 is energized to generate an alternating magnetic field, the electromagnetic ring 146 generates magnetism in turn along a circumferential direction, and the magnetic pole 147 deviates from a center due to the magnetic force and moves circumferentially along with the magnetic field. The movement of the magnetic pole 147 drives the distal end part of the water jet tube 120 to swing circumferentially, that is, the distal end of the water jet tube 120 makes a two-dimensional circumferential swing, thus driving the water jet nozzle 130 fixed at the distal end of the water jet tube 120 to move, and finally enabling the jet ejected by the water jet nozzle 130 to generate the reciprocating scanning movement. In this way, compared with the case of the driving motor, the example in Fig. 10 omits the transmission between the driving motor and the water jet tube by adopting the driving device including the electromagnetic ring and the magnetic pole, thereby reducing the assembly complexity of the driving device.

Figs. 11A-11B are schematic diagrams of a driving device for driving a water jet tube to rotate/revolve or swing according to some embodiments of the present disclosure.

In some examples, as shown in Figs. 11A and 11B, the driving device 140 includes a driving motor 101, an eccentric structure 102 and a second connector 103. The driving motor 101 includes an output shaft along the central axis of the outer tube 110, and the eccentric structure 102 is sleeved on the output shaft of the third motor 101. One end of the second connector 103 is connected with the eccentric structure 102, and the other end of the second connector 103 is sleeved outside the connecting part of the water jet tube 120 and forms a clearance fit with the connecting part, so that the driving motor 101 drives the eccentric structure 102 to move through the rotation of the output shaft, for example, to revolve around the central axis of the outer tube 110.

For example, the second connector 103 is a connecting rod, the eccentric structure 102 is sleeved on the output shaft of the driving motor 101 to form a tight fit, and the connecting rod is sleeved on the eccentric structure 102 to form a tight fit. For example, the driving motor 101 is a micro motor, which may refer to a motor with a diameter less than 160 mm or a rated power less than 750 mW. For example, the driving motor 101, the eccentric structure 102 and the second connector 103 are arranged in the shell of the handle (as a supporting body), and the handle is sleeved outside the connecting part of the outer tube 110 and fixedly connected with the connecting part of the outer tube 110.

For example, as shown in Fig. 11B, the medical water jet scalpel device 100 may also include a liquid supply connecting pipeline 170. For details, reference may be made to the above description, and will not be repeated here.

As shown in Figs. 11A and 11B, the handle further includes two fulcrum parts 104 respectively arranged at two sides of the second connector 103, and the two fulcrum parts 104 are also arranged in the shell of the corresponding handle. Therefore, the driving motor 101 is configured to drive the eccentric structure 102 to move, and then drive the second connector 103 to move, so that the second connector 103 partially contacts with the fulcrum part 104, that is, the fulcrum part 104 plays a role of limiting the second connector 103, thus restricting the proximal end of the water jet tube from circumferential movement when the second connector 103 rotates. Therefore, the proximal end part of the water jet tube is driven to swing circumferentially in two dimensions, so that the distal end part of the water jet tube swings circumferentially in two dimensions, that is, each point of the distal end part of the water jet tube is in translation movement rather than rotation/revolution. In this way, it has lower requirements for a sealing between the water jet tube 120 and the liquid supply connecting pipeline 170, and the structure can be simplified.

Therefore, in the embodiment of the present disclosure, the micro motor is used to drive the water jet tube to swing circumferentially in two dimensions, and the micro motor has smaller volume and weight, which makes it easier for the operator to control and less likely for the operator to feel tired when using the medical water jet scalpel device.

Fig. 12 is a schematic diagram illustrating components of a medical water jet scalpel system according to some embodiments of the present disclosure.

For example, as shown in Fig. 12, the medical water jet scalpel system includes a medical water jet scalpel device 100 and a host system 600, and the host system 600 includes a liquid supply unit 200 and a pressure pump 300. The liquid supply unit 200 is configured to provide the medical liquid. The medical water jet scalpel device 100 of the medical water jet scalpel system may be a medical water jet scalpel device 100 of any of the above examples, and the technical effect of the medical water jet scalpel system may refer to the above description related to the medical water jet scalpel device 100, which is not repeated here.

In some examples, the pressure pump 300 is configured to apply a pressure to the medical liquid, and the water jet tube 120 has a space for the medical liquid to circulate. Under the pressure of the pressure pump 300, the medical liquid can flow into the water jet tube 120, and then the medical liquid flows through an inner cavity of the water jet tube 120 and is ejected from the orifice of the water jet nozzle 130.

For example, the medical water jet scalpel device 100 is a disposable surgical device, which can be operated by a surgeon with one hand, and the water jet formed by the orifice of the water jet nozzle 130 of the medical water jet scalpel device 100 can efficiently complete the selective separation of soft tissues and elastic tissues.

In some examples, the liquid supply unit 200 includes a physiological saline bag, and the medical liquid includes physiological saline. This is only an example, and is not a limitation of the present disclosure. It can also be other suitable liquid supply units, such as a liquid reservoir, depending on actual situations, and will not be described in details here.

For example, in the example of Fig. 12, the medical water jet scalpel system further includes a wastewater reservoir 400 and a negative pressure aspirator 500. A gap space between an outer sidewall of the water jet tube and an inner sidewall of the outer tube 110 is used as an aspiration channel, the wastewater reservoir 400 is communicated with the outer tube 110, and the negative pressure aspirator 500 is connected to the wastewater reservoir 400 and provides a negative pressure capable of performing a suction function. In this way, waste liquid or tissue debris can be sucked, discharged and collected, which prevents from weakening the effect of selective separation of the medical water jet scalpel device. Moreover, since the outer tube 110 does not need to shake in the transverse direction, the outer tube 110 can be located at a position in a horizontal direction and a depth direction which is more conducive to negative pressure aspiration in a slit of a cutting edge, thereby improving the aspiration effect in the slit and obviously helping to improve the exposure depth and working efficiency of selective separation when using the medical water jet scalpel device.

In some examples, the medical water jet scalpel device 100 further includes an aspiration connecting tube 180. The aspiration connecting tube 180 is communicated with the proximal end of the outer tube 110, and the wastewater reservoir 400 is communicated with the outer tube 110 through the aspiration connecting tube 180. For example, the medical water jet scalpel device 100 may further include an aspiration adapter 160, and the communication between the aspiration connecting tube 180 and the outer tube 110 is realized through the aspiration adapter 160.

For example, the pressure pump 300 is a high-pressure pump with a working pressure of 1-10 MPa, which is only exemplary and not limited in the present disclosure.

In some examples, the host system 600 further includes a host part and the host part includes a control unit and a power unit. The host part is connected with the pressure pump 200 for controlling and driving the pressure pump 300, for example, a rotational speed may be adjusted. In some other embodiments, the pressure pump 300 itself has a power unit, or the pressure pump 300 is integrated with a control unit and a power unit.

Hereinafter, an operation method of the medical water jet scalpel system provided by some embodiments of the present disclosure will be described. The operation method includes the following steps: the liquid supply unit 200 supplies the medical liquid to the pressure pump 300; under the action of the control unit and the power unit, the pressure pump 300 pressurizes the medical liquid to the working pressure, then the medical liquid enters the handle 150a through the liquid supply connecting pipeline 170 and reaches the water jet nozzle 130 through the water jet tube 120; and a high-speed jet is generated from the orifices of the water jet nozzle 130 for ejecting, so as to realize the selective separation of soft tissues and elastic tissues in vivo by using the jet. For example, the selective separation of liver parenchyma and lumen-like tissues can be realized by using the jet.

The following points need to be clarified:
(1) The drawings of the embodiments of the present disclosure only refer to the structures to which the embodiments of the present disclosure relate, and other structures may refer to general designs.
(2) In case of no conflict, the embodiments of the present disclosure and the features in the embodiments may be combined with each other to obtain new embodiment(s).

The above description is only about specific implementations of the present disclosure, but the scope of protection of the present disclosure is not limited thereto. The scope of protection of the present disclosure should be subject to the scope of protection of the appended claims.

## Claims

1. A medical water jet scalpel device, comprising:
an outer tube;
a water jet tube extending through the outer tube and configured to input medical liquid, wherein a gap is provided between an outer wall of the water jet tube and an inner wall of the outer tube;
a water jet nozzle arranged at a distal end of water jet tube and configured to output the medical liquid; and
a driving device connected with at least part of the water jet tube, and configured to drive a distal end part of the water jet tube to swing reciprocally or revolve around a central axis of the outer tube.

2. The medical water jet scalpel device according to claim 1, wherein
a proximal end part of the water jet tube comprises a connecting part fixedly connected with at least part of the driving device, the distal end part of the water jet tube and the connecting part of the water jet tube are not coaxial with each other, so that the driving device drives the proximal end part of the water jet tube to rotate around a central axis of the water jet tube and enables the distal end part of the water jet tube to revolve around the central axis of the outer tube.

3. The medical water jet scalpel device according to claim 2, wherein
the water jet tube is integrally bent so that the distal end part and the connecting part are not coaxial with each other.

4. The medical water jet scalpel device according to claim 2, wherein
the distal end part of the water jet tube is configured as a bending part, and the distal end part has at least two segments which are not coaxial with each other, so that the distal end part and the connecting part are not coaxial with each other.

5. The medical water jet scalpel device according to claim 3 or 4, wherein
when the distal end part of the water jet tube abuts against an inner wall of a first side of the outer tube along a radial direction, the proximal end part of the water jet tube abuts against an inner wall of the other side of the outer tube opposite to the first side along the radial direction.

6. The medical water jet scalpel device according to claim 3 or 4, wherein
when the distal end part of the water jet tube abuts against an inner wall of a first side of the outer tube along a radial direction, the proximal end part of the water jet tube is arranged with a gap relative to an inner wall of the other side of the outer tube opposite to the first side along the radial direction.

7. The medical water jet scalpel device according to claim 1, wherein
the proximal end part of the water jet tube is configured to be fixed relative to the outer tube, and the driving device is configured to drive the distal end part of the water jet tube to swing reciprocally in one dimension along a radial direction of the water jet tube relative to the outer tube, or to swing circumferentially in two dimensions around the central axis of the outer tube.

8. The medical water jet scalpel device according to claim 3 or 4, further comprising a supporting body, wherein
the supporting body is sleeved outside a proximal end of the outer tube, and the supporting body is fixedly connected with the proximal end of the outer tube.

9. The medical water jet scalpel device according to claim 8, wherein
the supporting body comprises a handle, the handle is fixedly connected with the driving device, and the handle is configured to be driven by an external driving source to move in a direction of the central axis of the outer tube, so that the distal end part of the water jet tube moves in the direction of the central axis of the outer tube.

10. The medical water jet scalpel device according to claim 9, wherein
the driving device comprises a driving motor, and the driving motor is connected with the connecting part of the water jet tube to drive the proximal end part of the water jet tube to rotate around the central axis of the water jet tube.

11. The medical water jet scalpel device according to claim 10, wherein
the handle comprises a shell, the driving motor comprises a first motor and the first motor is arranged in the shell,
the driving device further comprises a driver connecting line, and the first motor is configured to obtain electric energy and a control signal through the driver connecting line, and
the first motor is sleeved outside the connecting part of the water jet tube and coaxially fixed with the water jet tube to drive the proximal end part of the water jet tube to rotate around the central axis of the water jet tube.

12. The medical water jet scalpel device according to claim 10, wherein
the handle comprises a shell, the driving motor comprises a second motor, the driving device further comprises a first connector, and the second motor and the first connector are arranged in the shell;
the first connector comprises a first gear and a second gear, and the first gear is meshed with the second gear; the first gear is fixedly connected with the connecting part of the water jet tube, and the second gear is connected with the second motor; and
the second motor is configured to drive the second gear to rotate to drive the first gear to rotate, so as to drive the proximal end part of the water jet tube to rotate around the central axis of the water jet tube.

13. The medical water jet scalpel device according to claim 10, further comprising a liquid supply connecting pipeline, wherein
one end of the liquid supply connecting pipeline is connected with a proximal end of the water jet tube, and the other end of the liquid supply connecting pipeline is configured to be connected with an output end of a pressure pump to input the medical liquid.

14. The medical water jet scalpel device according to claim 13, wherein
the handle comprises a shell, the driving motor is arranged outside the shell, and the driving motor is fixedly connected with the liquid supply connecting pipeline and configured to drive the liquid supply connecting pipeline to rotate so as to drive the proximal end part of the water jet tube to rotate around the central axis of the water jet tube.

15. The medical water jet scalpel device according to claim 7, further comprising a supporting body, wherein
the supporting body comprises a handle, and the handle comprises a shell, wherein the shell is sleeved outside a proximal end of the outer tube and fixedly connected with the proximal end of the outer tube;
the driving device comprises an electromagnetic ring and a magnetic pole, wherein the electromagnetic ring is arranged in the shell of the handle, the magnetic pole is fixedly arranged at a connecting part of the water jet tube, and a gap is provided between the magnetic pole and the electromagnetic ring, and the electromagnetic ring is configured to generate magnetism in turn along a circumferential direction by being energized, so as to attract the magnetic pole to move circumferentially and drive the distal end part of the water jet tube to swing circumferentially in two dimensions.

16. The medical water jet scalpel device according to claim 1, further comprising a supporting body, wherein
the driving device comprises a third motor, an eccentric structure and a second connector, and the supporting body comprises two fulcrum parts respectively arranged at two sides of the second connector,
the third motor comprises an output shaft along the central axis of the outer tube, the eccentric structure is sleeved on the output shaft of the third motor, one end of the second connector is connected with the eccentric structure, and the other end of the second connector is sleeved outside a connecting part of the water jet tube and is in clearance fit with the connecting part,
the third motor is configured to drive the eccentric structure to revolve around the central axis of the outer tube through a rotation of the output shaft, so as to drive the second connector to move, and
the second connector sequentially contacts with one of the two fulcrum parts to restrict the second connector from driving the connecting part to move circumferentially, so that the distal end part of the water jet tube swings circumferentially in two dimensions around the central axis of the outer tube.

17. A medical water jet scalpel system, comprising:
the medical water jet scalpel device according to any one of claims 1 to 16;
a liquid supply unit configured to provide the medical liquid; and
a pressure pump configured to apply a pressure to the medical liquid to input the medical liquid into the water jet tube.
